# EUROPÄISCHE PATENTANMELDUNG

(11) **EP 3 034 156 A1**
(43) Veröffentlichungstag der Anmeldung: **22.06.2016**
(21) Anmeldenummer: 15198371.5
(22) Anmeldetag: 08.12.2015
(51) Int. Cl.: B01D 53/26, B01D 53/28, C07B 63/00, C07D 233/00, C07C 51/44, F24F 3/14

(54) **VERFAHREN ZUM REINIGEN EINER IONISCHEN FLÜSSIGKEIT UND VERFAHREN ZUM ENTFEUCHTEN VON LUFT**

(30) Priorität: 18.12.2014 DE 102014226441
(71) Anmelder: Evonik Degussa GmbH, 45128 Essen (DE)
(72) Erfinder: ZEHNACKER, Olivier, 44263 Dortmund (DE); WILLY, Benjamin, 40211 Dortmund (DE); WANG, Xinming, Yamato-shi, 242-0003 Kanagawa-ken (JP); SCHNEIDER, Rolf, 63584 Gründau-Rothenbergen (DE); SCHNEIDER, Marc-Christoph, 63067 Offenbach (DE)

(57) **Zusammenfassung**

Ionische Flüssigkeiten der Struktur Q⁺A⁻, worin Q⁺ ein 1,3-Dialkylimidazoliumion ist, bei dem die Alkylgruppen unabhängig voneinander lineare C₁-C₄-Alkylgruppen sind und A⁻ das Anion einer Säure HA mit einem pKₐ von weniger als 3 ist, lassen sich durch Desorption von flüchtigen Verbindungen bei einer Temperatur von 100 bis 200 °C und einem Druck von maximal 100 mbar über einen Zeitraum von mindestens 0,1 h reinigen und für eine Entfeuchtung von Luft verwenden.

## Beschreibung

Die Erfindung betrifft ein Verfahren zum Reinigen einer ionischen Flüssigkeit und ein Verfahren zum Entfeuchten von Luft mit der gereinigten ionischen Flüssigkeit.

In Klimaanlagen für die Belüftung und Klimatisierung von Gebäuden oder Fahrzeugen muss die Luft in der Regel nicht nur gekühlt, sondern auch entfeuchtet werden, da die zu kühlende Luft oft eine so hohe Luftfeuchtigkeit aufweist, dass beim Kühlen auf die gewünschte Temperatur der Taupunkt unterschritten wird. In konventionellen Klimaanlagen entfällt deshalb ein großer Teil des Stromverbrauchs auf die Entfeuchtung der Luft.

Eine Möglichkeit, den Stromverbrauch von Klimaanlagen für Gebäude zu senken, ist die Entfeuchtung von Luft durch Adsorption oder Absorption von Wasser mit einem Trocknungsmedium und eine Regenerierung des mit Wasser beladenen Trocknungsmediums durch Erhitzen auf eine Temperatur, bei der das Wasser wieder desorbiert wird. Eine Absorption in einem flüssigen Absorptionsmedium hat dabei gegenüber einer Adsorption an einem festen Absorbens den Vorteil, dass die Lufttrocknung apparativ einfacher und mit weniger Trocknungsmedium durchgeführt werden kann und dass die Regenerierung des mit Wasser beladenen Trocknungsmediums einfacher mit Solarwärme durchgeführt werden kann.

Die in kommerziellen Klimaanlagen bisher als flüssige Absorptionsmedien eingesetzten wässrigen Lösungen von Lithiumbromid, Lithiumchlorid oder Calciumchlorid haben den Nachteil, dass sie gegenüber den üblicherweise in Klimaanlagen eingesetzten metallischen Werkstoffen korrosiv sind und deshalb die Verwendung von teuren speziellen Werkstoffen erfordern. Außerdem können mit diesen Lösungen Probleme durch eine Kristallisation von Salz aus dem Absorptionsmedium auftreten.

In Y. Luo et al., Appl. Thermal Eng. 31 (2011) 2772-2777 wird vorgeschlagen, an Stelle einer wässrigen Lösungen von Lithiumbromid die ionische Flüssigkeit 1-Ethyl-3-methylimidazoliumtetrafluoroborat zur Lufttrocknung einzusetzen.

In Y. Luo et al., Solar Energy 86 (2012) 2718-2724 wird für die Lufttrocknung als Alternative zu 1-Ethyl-3-methylimidazoliumtetrafluoroborat die ionische Flüssigkeit 1,3-Dimethyimidazoliumacetat vorgeschlagen.

In US 2011/0247494 A1 wird in Absatz [0145] die Verwendung von Trimethylammoniumacetat oder 1-Ethyl-3-methylimidazoliumacetat als flüssige Trocknungsmittel an Stelle von wässriger Lithiumchloridlösung vorgeschlagen. Beispiel 3 vergleicht für eine Reihe weiterer ionischer Flüssigkeiten die Wasseraufnahme aus feuchter Luft.

CN 102335545 A beschreibt wässrige Lösungen von ionischen Flüssigkeiten als Absorptionsmedien für eine Luftentfeuchtung. Die ionischen Flüssigkeiten können die Anionen [BF₄]⁻, [CF₃SO₃]⁻, [CH₃COO]⁻, [CF₃COO]⁻, [C₃F₇COO]⁻, [(CF₃SO₂)₂N]⁻, [(CH₃)PO₄]⁻, [C₄F₉SO₃]⁻, [(C₂F₅SO₂)N]⁻ und [(CF₃SO₂)₃C]⁻ enthalten.

Es wurde nun gefunden, dass kommerziell erhältliche ionische Flüssigkeiten in der Regel Verunreinigungen enthalten, die dazu führen, dass bei einer Entfeuchtung von Luft mit der ionischen Flüssigkeit geruchsintensive oder gesundheitsschädliche Stoffe in die entfeuchtete Luft gelangen. Es wurde außerdem gefunden, dass sich beim Desorbieren von Wasser aus ionischen Flüssigkeiten, die ein basisches Anion enthalten, wie zum Beispiel ein Carboxylation, geruchsintensive Zersetzungsprodukte bilden, die bei einer anschließenden Verwendung der ionischen Flüssigkeit zum Entfeuchten von Luft in die entfeuchtete Luft gelangen. Darüber hinaus wurde gefunden, dass sich durch eine Desorption von flüchtigen Verbindungen bei 100 bis 200 °C und einem Druck von weniger als 100 mbar aus einer ionischen Flüssigkeit mit einem nicht oder schwach basischen Anion eine gereinigte ionische Flüssigkeit herstellen lässt, mit der sich Luft entfeuchten lässt, ohne geruchsintensive oder gesundheitsschädliche Stoffe in die entfeuchtete Luft einzubringen.

Gegenstand der Erfindung ist deshalb ein Verfahren zum Reinigen einer ionischen Flüssigkeit, bei dem aus einer ionischen Flüssigkeit der Struktur Q⁺A⁻, worin Q⁺ ein 1,3-Dialkylimidazoliumion ist, bei dem die Alkylgruppen unabhängig voneinander lineare C₁-C₄-Alkylgruppen sind und A⁻ das Anion einer Säure HA mit einem pKₐ von weniger als 3 ist, bei einer Temperatur von 100 bis 200 °C und einem Druck von maximal 100 mbar über einen Zeitraum von mindestens 0,1 h flüchtige Verbindungen desorbiert werden.

Gegenstand der Erfindung ist außerdem ein Verfahren zum Entfeuchten von Luft, bei dem Luft mit einem Absorptionsmedium in Kontakt gebracht wird, das eine mit dem erfindungsgemäßen Verfahren gereinigte ionische Flüssigkeit der Struktur Q⁺A⁻ umfasst, worin Q⁺ ein 1,3-Dialkylimidazoliumion ist, bei dem die Alkylgruppen unabhängig voneinander lineare C₁-C₄-Alkylgruppen sind, A⁻ das Anion einer Säure HA mit einem pKₐ von weniger als 3 ist und kein Halogenidion ist und wobei eine Mischung aus 95 Gew.-% ionischer Flüssigkeit Q⁺A⁻ und 5 Gew.-% Wasser bei einer Temperatur von 80 °C einen Dampfdruck von weniger als 100 mbar aufweist.

Die erfindungsgemäßen Verfahren werden mit einer ionischen Flüssigkeit der Struktur Q⁺A⁻ durchgeführt. Q⁺ ist ein 1,3-Dialkylimidazoliumion, bei dem die Alkylgruppen unabhängig voneinander lineare C₁-C₄-Alkylgruppen sind. Geeignete 1,3-Dialkylimidazoliumionen sind 1,3-Dimethylimidazolium, 1-Ethyl-3-methylimidazolium, 1-Propyl-3-methylimidazolium, 1-Butyl-3-methylimidazolium, 1,3-Diethylimidazolium, 1-Ethyl-3-propylimidazolium, 1-Butyl-3-ethylimidazolium, 1,3-Dipropylimidazolium, 1-Butyl-3-propylimidazolium und 1,3-Dibutylimidazolium. Vorzugsweise ist Q⁺ ein 1,3-Dimethylimidazolium-Kation oder ein 1-Ethyl-3-methylimidazolium-Kation und besonders bevorzugt ein 1,3-Dimethylimidazolium-Kation. 1,3-Dialkylimidazoliumionen mit linearen Alkylgruppen haben gegenüber 1,3-Dialkylimidazoliumionen mit verzweigten Alkylgruppen den Vorteil, dass eine Dealkylierung während der erfindungsgemäßen Verfahren vermieden wird. 1,3-Dialkylimidazoliumionen mit C₁-C₄-Alkylgruppen haben gegenüber 1,3-Dialkylimidazoliumionen mit längeren Alkylgruppen den Vorteil, dass sie in einem Absorptionsmedium eine höhere Wasseraufnahmekapazität bei der Entfeuchtung von Luft erreichen.

Dier ionische Flüssigkeit der Struktur Q⁺A⁻ umfasst als Anion A⁻ das Anion einer Säure HA mit einem pKₐ von weniger als 3. Der pKₐ bezieht sich dabei auf eine wässrige Lösung der Säure HA bei 25 °C. Das Anion A⁻ kann das Anion einer organischen oder einer anorganischen Säure sein. Vorzugsweise ist das Anion A⁻ Hydrogensulfat, Methansulfonat, Methylsulfat, Ethylsulfat, Dihydrogenphosphat, Dimethylphosphat, Diethylphosphat oder Nitrat. Besonders bevorzugt ist Diethylphosphat das Anion A⁻. Durch die Verwendung eines Anions A⁻ einer Säure HA mit einem pKₐ von weniger als 3 lässt sich vermeiden, dass es bei der erfindungsgemäßen Reinigung und bei einer späteren Verwendung der ionischen Flüssigkeit zur Luftentfeuchtung zur Bildung von geruchsintensiven Verunreinigungen kommt, während mit ionischen Flüssigkeiten mit einem Anion einer schwächeren Säure, insbesondere mit einem Carboxylation, während der erfindungsgemäßen Reinigung und bei einer Verwendung der ionischen Flüssigkeit zur Luftentfeuchtung geruchsintensive Verunreinigungen entstehen können.

Bei dem erfindungsgemäßen Verfahren zum Reinigen einer ionischen Flüssigkeit werden aus der ionischen Flüssigkeit bei einer Temperatur von 100 bis 200 °C und einem Druck von maximal 100 mbar über einen Zeitraum von mindestens 0,1 h flüchtige Verbindungen desorbiert. Die Desorption von flüchtigen Verbindungen wird vorzugsweise bei einer Temperatur von 120 bis 180 °C und besonders bevorzugt von 140 bis 160 °C durchgeführt. Der Druck liegt dabei vorzugsweise bei 0,01 bis 20 mbar und besonders bevorzugt bei 0,01 bis 10 mbar. Die Desorption von flüchtigen Verbindungen wird vorzugsweise für eine Dauer von 0,5 bis 100 h, besonders bevorzugt 1 bis 10 h durchgeführt. Die Wahl von Temperatur, Druck und Dauer der Desorption erfolgt abhängig von der Menge und Art an flüchtigen Verbindungen in der eingesetzten ionischen Flüssigkeit, wobei für größere Mengen und für schwerer flüchtige Verbindungen eine höhere Temperatur, ein niedrigerer Druck und/oder eine längere Dauer gewählt werden. Geeignete Verfahrensbedingungen für die Desorption von flüchtigen Verbindungen können durch eine sensorische Prüfung der gereinigten ionischen Flüssigkeit auf Geruch, sowie durch Headspace-GC der gereinigten ionischen Flüssigkeit ermittelt werden.

Die Desorption von flüchtigen Verbindungen kann durch ein Vergrößern der Oberfläche der ionischen Flüssigkeit beschleunigt werden. Vorzugsweise wird dazu die ionische Flüssigkeit beim Desorbieren über eine Schüttung aus Füllkörpern oder über eine strukturierte Packung geleitet. Geeignet sind dazu alle Füllkörper und strukturierten Packungen, die dem Fachmann aus dem Stand der Technik für Destillationen und für Absorptionsprozesse bekannt sind. Alternativ kann die Desorption in einem Fallfilmapparat erfolgen. Als Fallfilmapparat eignen sich aus dem Stand der Technik für Destillationen bekannte Fallfilmverdampfer.

Die Desorption von flüchtigen Verbindungen kann außerdem beschleunigt werden, indem ein inertes Gas bei einem Druck von maximal 100 mbar durch die ionische Flüssigkeit geleitet wird oder im Gleichstrom oder Gegenstrom mit der ionischen Flüssigkeit durch eine Schüttung aus Füllkörpern, eine strukturierte Packung oder einen Fallfilmapparat geleitet wird. Als inerte Gase eignen sich Stickstoff, CO₂, Wasserdampf, Argon und Helium, wobei Stickstoff bevorzugt ist.

Die mit dem erfindungsgemäßen Verfahren gereinigte ionische Flüssigkeit kann zum Entfeuchten von Luft verwendet werden. Vorzugsweise erfolgt dies in einem Verfahren zum Entfeuchten von Luft, bei dem die Luft mit einem Absorptionsmedium in Kontakt gebracht wird, das eine ionische Flüssigkeit mit einer wie oben definierten Struktur Q⁺A⁻ umfasst, wobei eine Mischung aus 95 Gew.-% ionischer Flüssigkeit Q⁺A⁻ und 5 Gew.-% Wasser bei einer Temperatur von 80 °C einen Dampfdruck von weniger als 100 mbar aufweist. Das in Kontakt bringen kann in allen Apparaten erfolgen, die dem Fachmann für Gasabsorptionsverfahren mit flüssigen Absorptionsmitteln oder für die Lufttrocknung mit wässrigen Lösungen von Lithiumchlorid oder Lithiumbromid aus dem Stand der Technik bekannt sind. Geeignet sind zum Beispiel Apparate, in denen die Luft im Gleichstrom oder vorzugsweise im Gegenstrom mit dem Absorptionsmedium durch eine Schüttung aus Füllkörpern oder durch eine strukturierte Packung geleitet wird. Geeignet sind auch Apparate, in denen das Absorptionsmedium über von Luft umströmte Kühlrohre oder Kühlrippen rieselt oder abläuft. Vorzugsweise wird die Luft in einem Fallfilmapparat mit dem Absorptionsmedium in Kontakt gebracht. Durch die Verwendung eines Fallfilmapparats lässt sich vermeiden, dass die Luft Tröpfchen des Absorptionsmediums mitreißt.

Ionische Flüssigkeiten, die in einer Mischung mit 5 Gew.-% Wasser bei einer Temperatur von 80 °C einen Dampfdruck von weniger als 100 mbar aufweisen, lassen sich durch Routineexperimente auswählen, vorzugsweise aus ionischen Flüssigkeiten mit einem Anion aus der Gruppe Hydrogensulfat, Methansulfonat, Methylsulfat, Ethylsulfat, Dihydrogenphosphat, Dimethylphosphat, Diethylphosphat und Nitrat. Besonders geeignet sind die ionischen Flüssigkeiten 1,3-Dimethyimidazoluimhydrogensulfat, 1,3-Dimethyimidazoliummethansulfonat, 1,3-Dimethyimidazoliumethylsulfat, 1,3-Dimethyimidazoliumdiethylphosphat, 1,3-Dimethyimidazoliummnitrat, 1-Ethyl-3-methylimidazoliumhydrogensulfat, 1-Ethyl-3-methylimidazoliummethansulfonat, 1-Ethyl-3-methylimidazoliumethylsulfat, 1-Ethyl-3-methylimidazoliumdiethylphosphat und 1-Ethyl-3-methylimidazoliumnitrat.

In dem erfindungsgemäßen Verfahren zum Entfeuchten von Luft umfasst das Absorptionsmedium vor dem in Kontakt bringen mit Luft vorzugsweise mindestens 80 Gew.-% und besonders bevorzut mehr als 85 Gew.-% ionische Flüssigkeit der Struktur Q⁺A⁻. Vorzugsweise beträgt der Gesamtgehalt an ionischen Flüssigkeiten der Struktur Q⁺A⁻ und an Wasser in dem Absorptionsmedium mehr als 90 Gew.-%, besonders bevorzugt mehr als 98 Gew.-%.

Das bei dem Entfeuchten von Luft mit Wasser beladene Absorptionsmedium kann durch Verdampfen von Wasser wieder regeneriert und erneut zum Entfeuchten von Luft eingesetzt werden. Das mit Wasser beladene Absorptionsmedium wird dazu vorzugsweise erhitzt, vorzugsweise auf eine Temperatur von 70 bis 120 °C und das verdampfte Wasser wird kondensiert oder mit einem Luftstrom abgeführt. Das Kondensieren von Wasser kann durch Kühlen mit Wasser oder mit Luft bewirkt werden. Vorzugsweise wird das Verdampfen von Wasser in einem Fallfilmverdampfer durchgeführt und das verdampfte Wasser mit einem Luftstrom abgeführt, der besonders bevorzugt ein Abluftstrom aus einem klimatisierten Gebäude oder Fahrzeug ist.

Mit dem erfindungsgemäßen Verfahren lässt sich Luft mit geringen Mengen an Absorptionsmedium in einem Maß entfeuchten, wie es für den Betrieb einer Klimaanlage erforderlich ist, ohne dass es zu Problemen mit einer Kristallisation von Absorptionsmittel aus dem Absorptionsmedium oder durch eine Abgabe von gesundheitsschädlichen oder geruchsintensiven Stoffen aus dem Absorptionsmedium in die entfeuchtete Luft kommt.

### Beispiele

Aus den ionischen Flüssigkeiten wurden an einem Rotationsverdampfer bei einer Temperatur von 140 °C und einem Druck von 10 mbar über einen Zeitraum von 24 h flüchtige Verbindungen desorbiert. Die ionischen Flüssigkeiten wurden vor und nach der Behandlung sensorisch auf Geruch und durch Headspace-GC auf flüchtige Verunreinigungen untersucht. Für die Headspace-GC-Analyse wurde die ionische Flüssigkeit in einem Headspace-Probebehälter für 20 min auf 70 °C erhitzt bevor die Luft über der ionischen Flüssigkeit gaschromatographisch analysiert wurde. Die sensorische Bewertung des Geruchs und die mit Headspace-GC in der Luft nachgewiesenen Stoffe sind in den Tabellen 2 und 3 angeführt. Mit Ausnahme der ionischen Flüssigkeit von Beispiel 4, die von der Firma lolitec bezogen wurde, wurden die ionischen Flüssigkeiten durch Kondensation von Glyoxal, Formaldehyd, Methylamin und der in Tabelle 1 angeführten Säure im Molverhältnis 1:1:2:1 nach dem in WO 2009/074535 beschrieben Verfahren hergestellt. In Beispiel 6 wurde während des Versuchs der größte Teil der ionischen Flüssigkeit zu 1-Methylimidazol und Methylacetat zersetzt.

**Tabelle 1**

| Untersuchte ionische Flüssigkeiten | | |
|---|---|---|
| Beispiel | Ionische Flüssigkeit | Synthese bzw. Hersteller |
| 1 | 1,3-Dimethyimidazoliumhydrogensulfat | Kondensation mit H₂SO₄ |
| 2 | 1,3-Dimethyimidazoliummethansulfonat | Kondensation mit CH₃SO₃H |
| 3 | 1,3-Dimethylimidazoliumnitrat | Kondensation mit HNO₃ |
| 4 | 1-Ethyl-3-methylimidazoliumdiethylphosphat | lolitec |
| 5 | 1,3-Dimethyimidazoliumdiethylphosphat** | Kondensation mit (EtO)₂P(O)OH |
| 6* | 1,3-Dimethyimidazoliumacetat | Kondensation mit Essigsäure |

| | | |
|---|---|---|
| *nicht erfindungsgemäß ** enthält Ethylhydrogenphosphat | | |

**Tabelle 2**

| Ungereinigte ionische Flüssigkeit | | |
|---|---|---|
| Beispiel | Sensorische Bewertung des Geruchs | Flüchtige Stoffe im Headspace-GC |
| 1 | Fischig | Nicht bestimmt |
| 2 | Fischig | Nicht bestimmt |
| 3 | Fischig | Nicht bestimmt |
| 4 | Schwach | Acetonitril, Dichlormethan |
| 5 | Stark fischig | 1,2-Diaminoethan, Trimethylamin, Ethanol, Aceton, 2-Aminobutan, Triethylphosphat |
| 6* | Stark fischig | 1,2-Diaminoethan, Methylacetat, Essigsäure |

| | | |
|---|---|---|
| *nicht erfindungsgemäß | | |

**Tabelle 3**

| Gereinigte ionische Flüssigkeit | | |
|---|---|---|
| Beispiel | Sensorische Bewertung des Geruchs | Flüchtige Stoffe im Headspace-GC |
| 1 | Schwach, süßlich | keine |
| 2 | Schwach, süßlich | Ethanol |
| 3 | Schwach, fischig | keine |
| 4 | Schwach | Ethanol, Triethylphosphat |
| 5 | Schwach, süßlich | Ethanol, Triethylphosphat |

Die Beispiele zeigen, dass sich mit dem erfindungsgemäßen Verfahren die ionischen Flüssigkeiten so reinigen lassen, dass sie im Gegensatz zu den ungereinigten ionischen Flüssigkeiten bei einer Entfeuchtung von Luft mit der ionischen Flüssigkeit keine gesundheitsschädlichen oder geruchsintensiven Stoffe an die Luft abgeben.

## Patentansprüche

1. Verfahren zum Reinigen einer ionischen Flüssigkeit, **dadurch gekennzeichnet, dass** aus einer ionischen Flüssigkeit der Struktur Q⁺A⁻, worin Q⁺ ein 1,3-Dialkylimidazoliumion ist, bei dem die Alkylgruppen unabhängig voneinander lineare C₁-C₄-Alkylgruppen sind und A⁻ das Anion einer Säure HA mit einem pKₐ von weniger als 3 ist, bei einer Temperatur von 100 bis 200 °C und einem Druck von maximal 100 mbar über einen Zeitraum von mindestens 0,1 h flüchtige Verbindungen desorbiert werden.

2. Verfahren nach Anspruch 1, **dadurch gekennzeichnet, dass** A⁻ ausgewählt ist aus Hydrogensulfat, Methansulfonat, Methylsulfat, Ethylsulfat, Dihydrogenphosphat, Dimethylphosphat, Diethylphosphat und Nitrat.

3. Verfahren nach Anspruch 1 oder 2, **dadurch gekennzeichnet, dass** Q⁺ ein 1,3-Dimethyimidazolium-Kation oder ein 1-Ethyl-3-methylimidazolium-Kation ist.

4. Verfahren nach einem der Ansprüche 1 bis 3, **dadurch gekennzeichnet, dass** das Desorbieren über einen Zeitraum von 0,5 bis 100 h erfolgt.

5. Verfahren nach einem der Ansprüche 1 bis 4, **dadurch gekennzeichnet, dass** die ionische Flüssigkeit beim Desorbieren über eine Schüttung aus Füllkörpern oder über eine strukturierte Packung geleitet wird.

6. Verfahren nach einem der Ansprüche 1 bis 4, **dadurch gekennzeichnet, dass** das Desorbieren in einem Fallfilmapparat erfolgt.

7. Verwendung einer mit einem Verfahren gemäß einem der Ansprüche 1 bis 6 gereinigten ionischen Flüssigkeit zum Entfeuchten von Luft.

8. Verfahren zum Entfeuchten von Luft, bei dem Luft mit einem Absorptionsmedium umfassend eine ionische Flüssigkeit der Struktur Q⁺A⁻ in Kontakt gebracht wird, **dadurch gekennzeichnet, dass**
Q⁺ ein 1,3-Dialkylimidazoliumion ist, bei dem die Alkylgruppen unabhängig voneinander lineare C₁-C₄-Alkylgruppen sind,
A⁻ das Anion einer Säure HA mit einem pKₐ von weniger als 3 ist und kein Halogenidion ist, eine Mischung aus 95 Gew.-% ionischer Flüssigkeit Q⁺A⁻ und 5 Gew.-% Wasser bei einer Temperatur von 80 °C einen Dampfdruck von weniger als 100 mbar aufweist,
und die ionische Flüssigkeit Q⁺A⁻ vor dem in Kontakt bringen mit Luft mit einem Verfahren gemäß einem der Ansprüche 1 bis 6 gereinigt wurde.

9. Verfahren nach Anspruch 8, **dadurch gekennzeichnet, dass** die ionische Flüssigkeit ausgewählt ist aus 1,3-Dimethyimidazoluimhydrogensulfat, 1,3-Dimethyimidazoliummethansulfonat, 1,3-Dimethyimidazoliumethylsulfat, 1,3-Dimethyimidazoliumdiethylphosphat, 1,3-Dimethyimidazoliummnitrat, 1-Ethyl-3-methylimidazoliumhydrogensulfat, 1-Ethyl-3-methylimidazoliummethansulfonat, 1-Ethyl-3-methylimidazoliumethylsulfat, 1-Ethyl-3-methylimidazoliumdiethylphosphat und 1-Ethyl-3-methylimidazoliumnitrat.

10. Verfahren nach Anspruch 8 oder 9, **dadurch gekennzeichnet, dass** das Absorptionsmedium vor dem in Kontakt bringen mit der Luft mindestens 80 Gew.-% ionische Flüssigkeit der Struktur Q⁺A⁻ umfasst.
